Europäisches Patentamt

(19) European Patent Office

Office européen des brevets

(11) Publication number: **0 289 449**
**A1**

## (12) EUROPEAN PATENT APPLICATION

(21) Application number: 88730099.4

(22) Date of filing: 28.04.88

(51) Int. Cl.⁴: **A 61 F 2/16**

(30) Priority: 01.05.87 US 44753    21.05.87 US 52400
29.06.87 US 67163    30.07.87 US 79509

(43) Date of publication of application:
02.11.88 Bulletin 88/44

(84) Designated Contracting States:
AT BE CH DE ES FR GB GR IT LI LU NL SE

(71) Applicant: **Anis, Aziz Y.**
**7531 North Hampton**
**Lincoln Nebraska 68506 (US)**

(72) Inventor: **Anis, Aziz Y.**
**7531 North Hampton**
**Lincoln Nebraska 68506 (US)**

(74) Representative: **Huber, Arnulf et al**
**Uexküll & Stolberg, Patentanwälte Beselerstrasse 4**
**D-2000 Hamburg 52 (DE)**

(54) **Posterior chamber lens.**

(57) An intraocular lens implant comprising a flexible, substantially ring-shaped position fixation member (20) having a lens body (14) positioned centrally therein. The lens body is connected to the position fixation member by either rim portions (22, 24) or posts which have a notch formed therein for receiving one end of the incision through which the implant will be inserted. The notch permits the implant to be inserted in an incision shorter than the diameter of the implant. The method of implanting the implant is also described.

FIG. 1

## Description

### POSTERIOR CHAMBER LENS

This invention relates to a posterior chamber lens.

The human eye is a very complex organ comprising numerous interacting elements which gather, focus, and transmit light rays to nerve endings which eventually transmit the information to the brain for image perception. The eye includes a natural crystalline lens of avascular tissue, the transparency of which depends upon the critical regularity of its fibers and the balance of its chemical constituents. Obviously, there are enumerable factors which may interfere with lens makeup and thereby affect its transparent character. No matter what the reason, a condition of opacity in the lens, commonly called cataract, reduces the visual performance of the eye. When the visual performance is reduced to an unacceptable level, surgical cataract extraction becomes a necessity.

An eye without a lens, a condition called aphakia, is obviously defective from an optical point of view in as much as it cannot properly refract incident light rays. Aphakic correction may be accomplished in three ways:

(1) thick eye glasses worn in front of the eye;
(2) contact lenses worn on the eye, or
(3) artificial intraocular lens implant within the eye.

It is this latter procedure with which the instant invention is concerned.

The structure and procedure of installing an intraocular lens is very critical because of the elements which make up the eye are extremely sensitive and subject to irreparable damage. Numerous experimental lens designs have been abandoned through the years because they caused corneal damage and other manifestations of intraocular irritation. For example, in the late 1940's and early 1950's, H. Ridley conducted clinical experiments with an artificial intraocular lens which included a lens portion having foot-like projections extending radially away therefrom. This device was placed in the posterior chamber with the feet extending between the ciliary processes and the base of the iris. The lens proved positionally unstable and resulted in unsatisfactory amounts of irritation.

U.S. Patent 3,866,249 discloses a posteriorly positioned prosthetic lens which was a multiplicity of forwardly projecting prongs. During surgical implantation, the prongs are extended through the iris to anchor the lens in position. While this arrangement certainly maintains positional integrity, it, too, has distinct disadvantages. The great number of prongs extending through and over the iris promote undesirable irritational characteristics, and the numerous fixation points also have a tendency to distort the iris by pulling on it in numerous directions.

U. S. Patents 3,925,825, 3,913,148, and 3,922,728 teach a prosthetic lens structure which is, in one way or another, less than desirable in construction and use. In an effort to remedy the problems associated with the prior art lens implants, applicant previously has been granted U. S. patents 4,143,427, 4,166,293 and 4,251,887. Other recent developments relating to implant lens may be found in U. S. patents 4,316,293 and 4,340,979.

A problem associated with the implantation of posterior chamber lenses is that opacification of the posterior capsule occurs through cellular migration onto the posterior capsule. A further problem associated with the prior art lenses is that the fixation elements do not engage substantially the entire capsular equator when the lens is implanted.

It is a task of the invention to provide an improved flexible posterior chamber lens.

In accordance with the invention, a posterior chamber lens includes a ring-shaped member extending around a centrally positioned lens body with at least one commecting member extending between the lens body and the fixation member. The fixation member is ring-shaped, extends around a centrally positioned lens and may be compressed relative to the centrally positioned lens body to enable the implant to be inserted into the capsular bag, wherein remains in place even if pressure or force is inadvertently applied to one portion of the lens.

A maximum contact of the position fixation element is provided by including at least one loop but preferably a pair of opposing compressible loops each of which extend around the lens body for greater than 180 degrees. In one embodiment of the invention, a posterior chamber lens is provided with a convex rear face which engages the posterior capsule to stretch the same rearwrdly so that cellular migration onto, and the opacification of the posterior capsule is substantially reduced or eliminated. In this or other embodiments, the flexible posterior chamber lens is of one-piece construction.

The invention will be better understood from the following examples when illustrated by the following drawings in which :

Figure 1 is an elevational view of one form of the invention with the broken lines indicating various positions of deflection of the position fixation member;

Figure 2 is an end view of the embodiment of Figure 1:

Figure 3 is a view similar to Figure 1 except that a modified form of the invention is disclosed:

Figure 4 is an end view of the embodiment of Figure 3;

Figure 5 is a view similar to Figures 1 and 3 except that a further modified form of the invention is illustrated.

Figure 6 is an elevational view of another embodiment of the invention:

Figure 7 is an end view of the embodiment of Figure 6:

Figure 8 is an end view of a further embodiment of the invention:

Figure 9 is an end view of the lens of Figure 6:

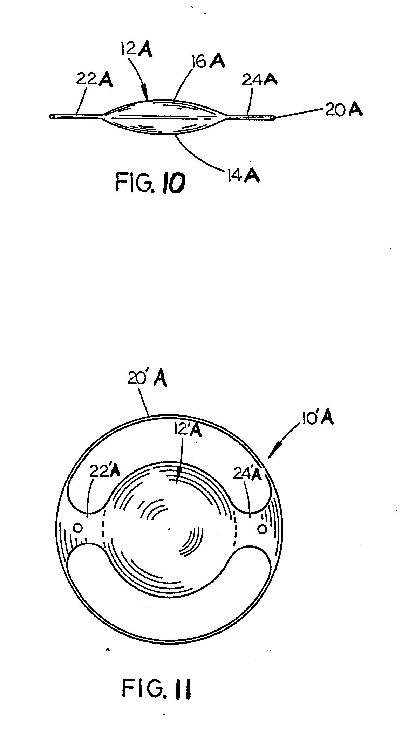

Figure 10 is a sectional view of the embodiment of Figure 6:

Figure 11 is an elevational view of a modified form of the embodiment of Figure 6:

Figure 12 is a perspective view of another embodiment of the invention:

Figure 13 is an elevational view of the embodiment of Figure 12:

Figure 14 is an end view of a modified form of the embodiment of Figure 12:

Figure 15 is an end view of a still further modified form of the embodiment of Figure 12:

Figure 16 is an end view of the embodiment of Figure 13:

Figure 17 is a plan view illustrating an incision being created during the implantation process:

Figure 18 is a view similar to Figure 17 but which illustrates the implant being implanted:

Figure 19 is a view similar to Figures 17 and 18 but which illustrates the lens being further implanted:

Figure 20 is a view illustrating the lens implant after it has been implanted:

Figure 21 is an elevational view of a modified form of the invention:

Figure 22 is an elevational view of the invention with the broken lines indicating various positions of deflection of the position fixation member; and

Figure 23 is an end view of the embodiment of Figure 22.

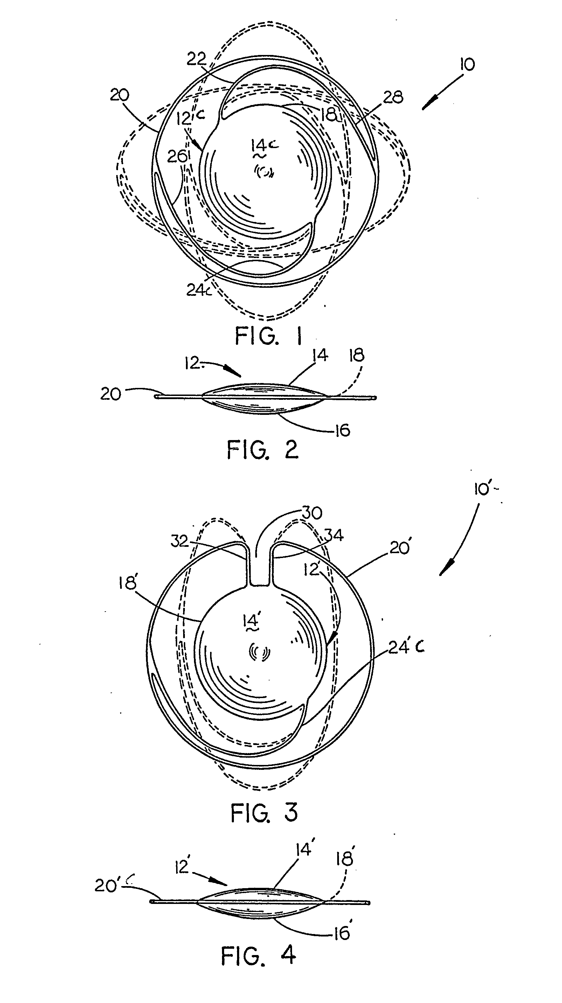

As shown in Figure 1, a posterior chamber lens implant which may be implanted in the eye after the natural lens of the eye has been removed includes a flexible, substantially ring-shaped position fixation member that extends around a lens body. The lens body has a diameter less than the position fixation element.

At least one flexible connection means connects the lens body with the position fixation member to cause the lens body to be centrally positioned with respect to the implant. The means of connecting the lens body to the fixation member also permits the fixation member to be compressed relative to the lens body to facilitate insertion of the implant into the eye. In a modified version of the invention, a pair of spaced-apart posts are substituted for one of the connecting members.

The lens implant of this invention is referred to generally by the reference numeral 10. Lens implant 10 includes a disc-shaped lens body 12 which may either be of the convex-plano or convex-convex configuration. For purposes of description, lens body 12 will be described as having a front face 14, back face 16 and peripheral edge 18.

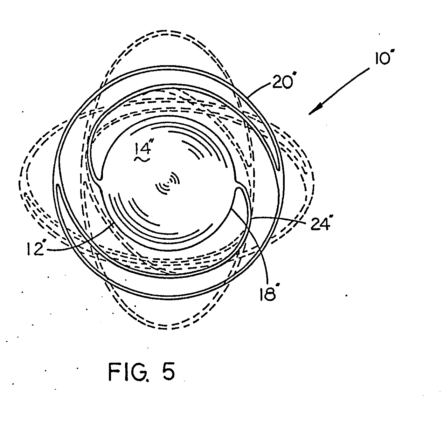

A ring-shaped position fixation member 20 is positioned outwardly of the lens body 12 and is connected thereto by a pair of elongated curved connecting members 22 and 24 which extend oppositely from the lens body 12. As shown by the drawings, each of the members 22 and 24 have a portion thereof which is generally concentrically positioned with respect to the fixation member 20 and which is referred to generally by the reference numerals 26 and 28 respectively. Preferably, lens body 12 has a diameter of 6 millimeters but can be between 4.0 and 8 millimeters. Preferably, the diameter of the ring forming fixation member 20 is 10.5 millimetes but the same can vary between 8.0 millimeters and 13.0 millimeters. Preferably, the diameter of a cross-section through the fixation member 20 is 0.15 millimeters. The construction of the lens implant 10 is such that the ring-shaped fixation member 20 will engage the capsular bag for 360 degrees. The connecting members 22 and 24 aid in centrally positioning the lens body 12 with respect to the fixation member 20. The construction of the lens implant 10 is such that the fixation member 20 may be compressed towards the lens body 12, 12 illustrated by the broken lines in Figure 1, 5o enable the implant 10 to be inserted into the capsular bag.

In the embodiment shown in Figures 3 and 4, the fixation member 20′ does not extend completely around the lens body 12′ but is provided with a slight gap referred to generally by the reference numeral 30. As shown in Figures 3 and 4, a pair of posts 32 and 34 extend radially outwardly from lens body 12′ and are connected to the substantially ring-shaped fixation member 20′. A single curved connecting member 24′ connects lens body 12 with the member 20′ substantially opposite the posts 32 and 34 as seen in the drawings. In the embodiment of Figures 3 and 4, the fixation member 20′ does engage the structure of the eye for almost 360 degrees. The posts 32 and 34 and the connecting member 24′ aid in centrally positioning the lens body 12′ with respect to the fixation member 20′. The construction of the lens implant of Figures 3 and 4 is substantially the same as the preferred embodiment of this invention in that it does permit the fixation member 20′ to be compressed relative to the lens body 12′ for insertion into the capsular bag as illustrated by broken lines in Figure 3.

In all of the embodiments, the lens may be inserted into position within the eye since there is not a top or bottom portion of the lens implant. The 360 degree engagement of the fixation member 20 with the eye positively ensures that the lens implant will remain in position and will not become inadvertently dislodged. The lens implant of Figures 3 and 4 functions similarly to that of the embodiment previously described.

In the embodiment shown in Figure 5, the fixation member 20″ extends completely around the lens body 12″ and is connected thereto by a pair of elongated curved connecting members 22″ and 24″ which extend oppositely from lens body 12″. Essentially, connecting member 24″ estends from the three o'clock position to the nine o'clock position while connecting member 22″ extends from approximately the nine o'clock position to the three o'clock position. The embodiment of Figure 5 is substantially the same as that shown in Figures 1 and 2 except that the connecting members are slightly longer and have a longer portion of their length concentric to the periphery of the lens body. In all of the embodiments, the fixation members may dwell in the same plane as the lens body as illustrated in the drawings or they may be offset therefrom.

In another embodiment of lens implant a pair of closing opposing connectors extend from the lens body to the position fixation member. The position fixation member may dwell in a single plane or may be concave shaped. The lens may be positioned on either the posterior or anterior side of the position fixation element. The lens body may assume any convenient configuration.

In this embodiment as in others: (1) the lens implant 10A includes a disc-shaped lens body 12A which may be of the convex-plano, convex-convex, or any other convenient configuration; (2) lens body 12A has a front or anterior face 14A, back or posterior face 16A and peripheral edge 18A; (3) a ring-shaped position fixation member 20A is positioned outwardly of the lens body 12A. The fixation member 20A is connected thereto by a pair of opposing posts 22A and 24A which extend oppositely from the lens body 12A. Preferably, lens body 12A has a diameter of 6 millimeters but the same can be between 4.0 and 8.0 millimeters. Preferably, the diameter of fixation member 20 is 10.5 millimeters but the same can vary between 8.0 millimeters and 13.0 millimeters. Preferably, the diameter of the fixation member 20 is 0.15 millimeters.

The construction of the lens implant 10A is such that the ring-shaped fixation member 20A engages the capsular bag for 360 degrees. The connecting posts 22A and 24A aid in centrally positioning the lens body 12A with respect to the fixation member 20A and adds stability to the lens implant. The construction of the lens implant 10A is such that the fixation member 20A may be compressed towards the lens body 12A between the connecting posts 22A and 24A.

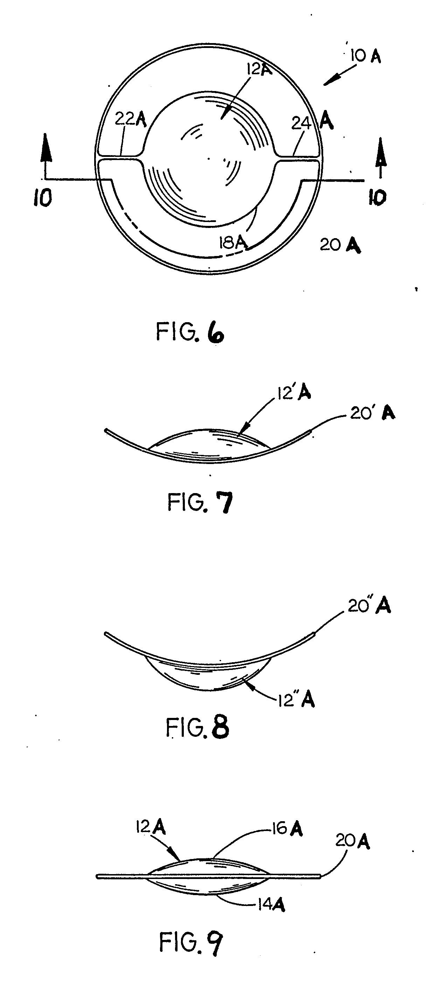

Figures 7 and 8 illustrate the fact that the fixation member 20'A may be curved as desired. Figures 7 and 8 also illustrate that the lens body 12'A may be positioned posteriorly of the fixation member 20'A or anteriorly of the position fixation member 20'.

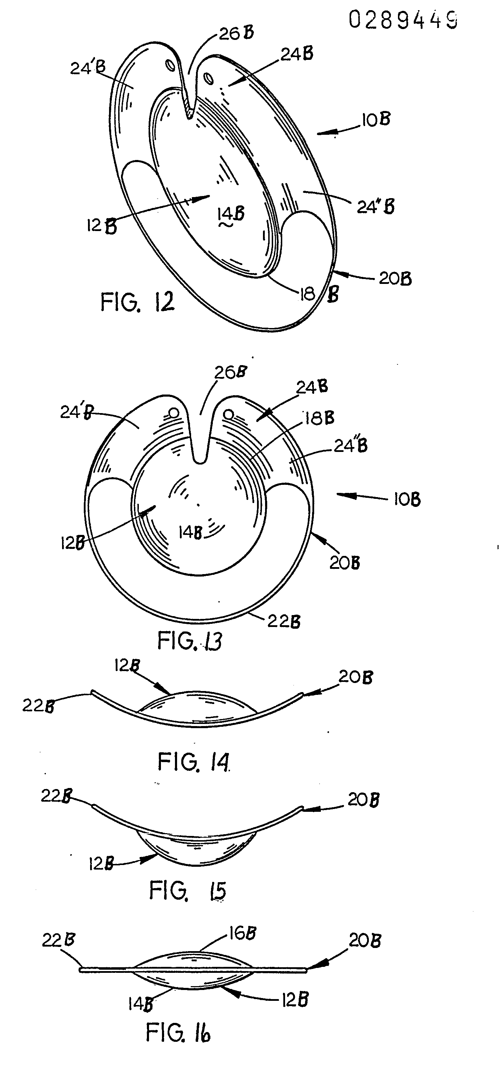

Figure 11 illustrates a modification of the invention and is referred to by the reference numeral 10'A. Lens implant 10'A includes a disc-shaped lens body 12'A which may be of the convex-plano, convex-convex, or any other convenient configuration. Lens implant 10'A is identical to lens implant 10A except that the posts 22'A and 24'A have a greater width than the posts 22A and 24A. By increasing the width of the posts 22'A and 24'A, it is believed that greater stability of the lens implant is achieved. During implantation, the fixation member 20'A will be compressed towards the lens body 12'A and the posts 22'A and 24'A prevent the fixation member 20'A from "bulging out" in the vicinity of the posts.

In all of the embodiments, the lens may be inserted in any position within the eye since there is not a top or bottom portion of the lens implant. The 360 degree engagement of the fixation member 20A with the eye positively ensures that the lens implant will remain in position and will not become inadvertently dislodged.

In another embodiment of the invention, a cut-out portion or notch extends into the lens implant to facilitate the insertion of the implant into the eye through an incision. During the installation, the lens implant is inserted into and through the incision so that the notch "receives" one end of the incision so that the lens implant may be inserted through a shorter incision than normally possible. Once the implant has been so positioned, the implant is rotated about the notch to position the implant within the eye. A portion of the position fixation member can be compressed towards the lens body to enable the implant to be properly positioned. When positioned within the eye, there is a substantially 360 degree engagement of the lens with the eye to positively maintain the lens implant in position.

In this embodiment, lens implant 10B includes a disc-shaped lens body 12B which may either be of the convex-plano or convex-convex or other conventional configuration as desired. For purposes of description, lens body 12B will be described as having a front or anterior face 14B, back or posterior face 16B and peripheral edge 18B.

A ring-shaped position fixation member 20B is positioned outwardly of the lens body 12B as illustrated in the drawings. As seen, fixation member 20B includes a loop portion 22B which extends from rim portion 24B. Rim portion 24B is provided with a notch or recess 26B formed therein which extends thereinto as seen in Figures 12 and 13, the inner end of the recess 26B extends into the lens body 12B.

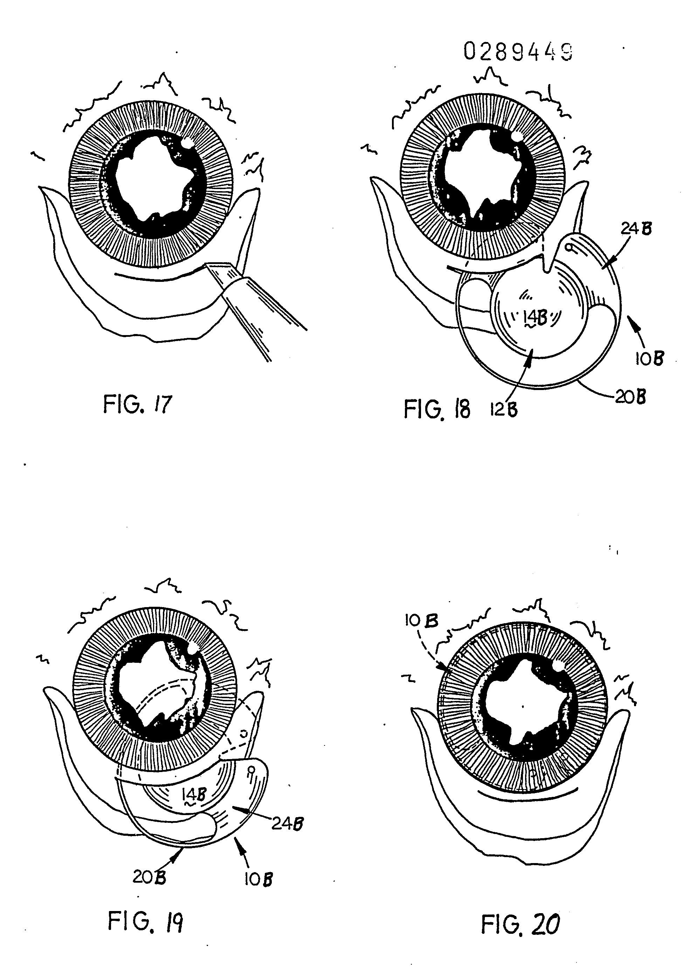

The lens implant of this invention is inserted or implanted as follows. As seen in Figure 17, an incision is created with the incision being shorter than would normally be required. As seen in Figure 18, the implant is inserted into the incision so that the notch 26B receives one end of the incision which permits the implant to be implanted through a shorter incision than normally required. The implant 10B is rotated or pivoted about that end of the incision which is received by the notch 26B and the implant 10B is pivoted or rotated utilizing the one end of the incision as a fulcrum or pivot point. As the lens implant 10B is rotated from the position of Figure 18 to the position of Figure 19, the fixation member 20B is compressed as illustrated in Figure 19. Rotation of the lens implant is continued until the entire implant is received within the capsular bag. Figure 20 illustrates the lens implant in its implanted condition.

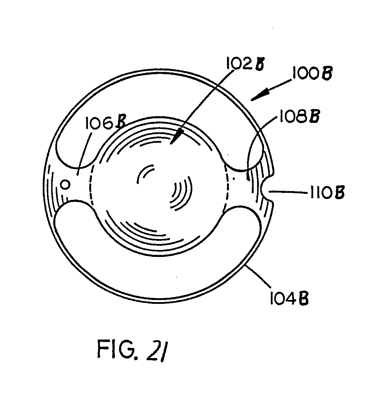

Figure 21 illustrates a modification of the invention and is referred to generally by the reference numeral 100B. Lens implant 100B includes a disc-shaped lens body 102B which may bed of a convex-plano, convex-convex, or any other convenient configuration. Lens implant 100B is provided with a ring-shaped position fixation member 104B extending therearound which is connected to the lens body 102B by a pair of opposed posts 106B and 108B as illustrated in Figure 21. The lens implant is provided with a notch 110B which extends into post 108B although the notch 110 could extend into the post 106B if desired. Notch 110B performs the same function as notch 26B is the embodiment described in Figures 12-20. During implantation, notch 110B in implant 100B would receive one end of the incision in an identical fashion to that just described.

Thus it can be seen that a novel lens implant has been provided which enables the implant to be inserted through a shorter incision than normally required due to the fact that the notch 26B or notch 110B is provided and is adapted to receive one end of the incision as previously described.

The substantially 360 degree engagement of the lens implant with the interior of the eye ensures that the implant will remain in position even if accidentally jarred or struck. Thus it can be seen that the implant of this invention accomplishes at least all of its stated objectives.

In still another embodiment, a lens implant comprises a centrally positioned lens body having a pair of opposing loop members secured thereto and extending therearound. One of the loop members extends substantially tangentially from the lens body at approximately three o'clock and extends around the lens body and in a spaced-apart relationship with respect thereto with the other end of the loop member being connected to the lens body at approximately three o'clock. The other loop member extends substantially tangentially from the lens body at approximately nine o'clock and extends around the lens body in a clockwise direction and in a spaced-apart relationship thereto with the other end of the other loop member being connected to the lens body at approximately nine o'clock. The means of connecting the loop members to the lens body permits the loop members to be compressed relative to the lens body to facilitate insertion of the implant into the eye.

In this embodiment lens implant 10E includes a disc-shaped lens body 12E which may either be of the conves-plano or convex-convex configuration. For purposes of description, lens body 12E will be described as having a front face 14E, back face 16E and peripheral edge 18E.

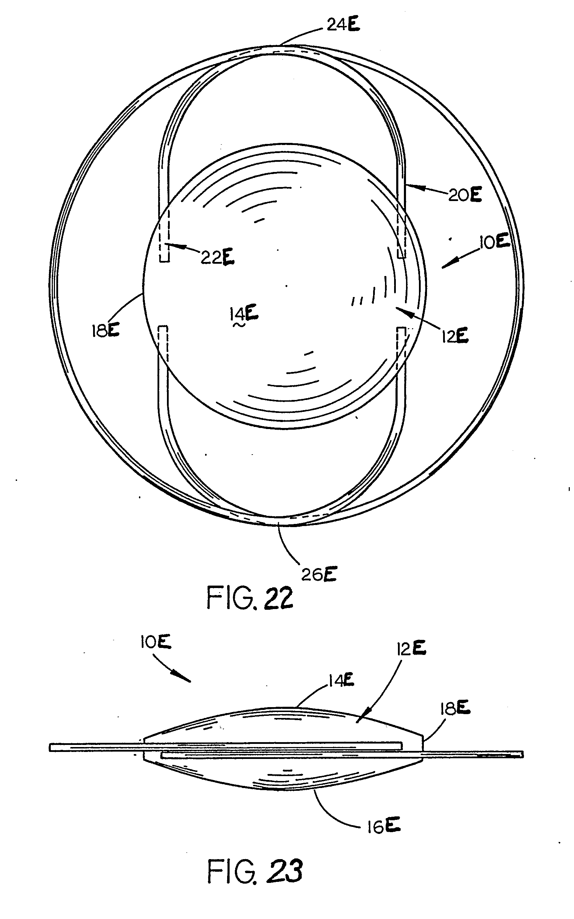

A pair of fixation members or loop members 20E and 22E are secured to the lens body 12E in the opposing relationship as illustrated in the drawings. One end of the fixation member 20E is secured to the lens body 12E and extends substantially tangentially therefrom at approximately the three o'clock position. Loop member 20E extends around the lens body in a counterclockwise manner as illustrated in Figure 22 and in a spaced-apart relationship with respect to the lens body 12E. The other end of the loop member 22E is secured to the lens body 12E at approximately the three o'clock position as best seen in Figure 32.

Loop member 22E has one end thereof secured to the lens body 12E at approximately the nine o'clock position and extends substantially tangentially therefrom as illustrated in Figure 31. Loop member 22E extends around the lens body 12E in a spaced-apart relationship and in a clockwise manner. The other end of loop member 22E is secured to the lens body 12E at substantially the nine o'clock position as seen in Figure 22. Preferably, loop members 20E and 22E dwell in slightly different plans so that they may intersect without substantial contact at 24E and 26E although the loop members 20E and 22E may be positioned in the same plane if desired which will require that the loop members "cross-over" at 24E and 26E. Further, the loop members 20E and 22E may be offset with respect to the lens body.

The primary feature of the instant invention is that the loop members cooperate with each other so that the loop members extend around the lens body for 360 degrees to enable the maximum contact of the fixation members within the eye. The lens may be inserted in any position within the eye since there is not a top or bottom portion of the lens implant. The 360 degree engagement of the fixation members with the eye positively ensures that the lens implant will remain in position and will not become inadvertently dislodged.

The construction of the lens implant 10E is such that the fixation members 20E and 22E engage the capsular bag for 360 degrees. The loop members 20E and 22E aid in centrally positioning the lens body 14E with respect to the eye. The construction of the lens implant 10E is such that the fixation members 20E and 22E may be compressed towards the lens body 14E, as illustrated by the broken lines in Figure 40, to enable the implant 10E to be inserted into the capsular bag.

## Claims

1. A one-piece intraocular lens implant (e.g. 10), having a lens body (14) and a fixation member, the lens body having a diameter less than said position fixation member and being positioned within said position fixation member, characterized in that the fixation member (20) is flexible and substantially ring-shaped and there is a flexible connection means (28) connecting said lens body (14) with said position fixation member (20).

2. The lens implant of claim 1 characterized in that said flexible connection means (28) comprises a pair of elongated members (28, 24) extending oppositely from said lens body (14), each of said elongated members (28, 24) having a substantial portion of its length which is substantially concentrically disposed with said position fixation member (20).

3. The lens implant of claims 1 or 2 characterized in that said flexible connection means (24, 28) comprises a pair of closely spaced-apart posts (32, 34) extending from said lens body (14) to said position fixation member (20) and at least one elongated curved member extending from said lens body to said position fixation element.

4. In intraocular lens implant according to any of claims 1-3 characterized in that said position fixation member (20) and said connection means (28) have a notch formed therein which extends inwardly thereinto for receiving one end of the incision through which the implant will be inserted.

5. A lens implant according to any of claims 1-4 characterized in that one loop members (20) has one end secured to said lens body (14) at approximately the three o'clock position and extends around the lens body in a spaced-apart relationship with respect thereto and has its other end secured to said lens body (14) at approximately the three o'clock position, the other of said loop members (20') having one end secured to said lens body (14) at approximately the nine o'clock position and extends around the lens body in a spaced-apart relationship with respect thereto and has its other end secured to said lens body at approximately the nine o'clock position.

6. A lens implant according to claim 5 in which said loop members dwell in substantially the same plane.

0289449

FIG. 1

FIG. 2

FIG. 3

FIG. 4

0289449

FIG. 5

0289449

FIG. 6

FIG. 7

FIG. 8

FIG. 9

0289449

FIG. 10

FIG. 11

0289449

FIG. 12

FIG. 13

FIG. 14.

FIG. 15

FIG. 16

0289449

FIG. 17

FIG. 18

FIG. 19

FIG. 20

0289449

FIG. 21

0289449

FIG. 22

FIG. 23

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | FR-A-2 530 457 (T. MAZZOCCO) * Figure 4; page 14, lines 11-20 * | 1,6 | A 61 F 2/16 |
| A | FR-A-2 515 956 (D. LEBUISSON) * Figure 1 * | 1,2,5 | |

TECHNICAL FIELDS SEARCHED (Int. Cl.4)

A 61 F

The present search report has been drawn up for all claims

| Place of search | Date of completion of the search | Examiner |
|---|---|---|
| THE HAGUE | 09-08-1988 | NEILL M.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P0401)